# NEW EUROPEAN PATENT SPECIFICATION

(11) **EP 1 962 797 B2**
(45) Date of publication and mention of the opposition decision: **29.05.2024**
(45) Mention of the grant of the patent: 28.10.2020
(21) Application number: 06829526.0
(22) Date of filing: 12.12.2006
(51) Int. Cl.: A61P 11/06, A61P 11/00, A61P 11/08, A61P 43/00, A61K 31/40, A61K 9/00

(54) **POWDER COMPOSITIONS FOR INHALATION**
PULVERZUSAMMENSETZUNGEN ZUR INHALATION
PRÉPARATIONS DE POUDRE POUR INHALATION

(30) Priority: 12.12.2005 GB 0525254
(43) Date of publication of application: 03.09.2008
(73) Proprietor: Jagotec AG, 4132 Muttenz (CH)
(72) Inventor: MUELLER-WALZ, Rudi, 79650 Schopfheim (DE)
(74) Representative: Summersell, Richard John
(86) International application number: PCT/EP2006/011941
(87) International publication number: WO 2007/068443

(56) References cited:
- WO-A-97/03649
- WO-A-2004/093848
- WO-A-2005/025540
- WO-A-2005/046636
- WO-A1-2005/046636
- WO-A2-02/43700
- WO-A2-2005/105043

## Description

The present invention is concerned with methods of forming powder formulations for use in dry powder inhalers (DPIs).

Powder formulations typically consist of a binary mixture of small drug particles having a mean aerodynamic diameter of about 1 to 10 microns, and a coarser carrier material for said drug particles. These components are usually blended together to form a so-called "interactive mixture" wherein the finer drug particles are strongly adhered to the carrier particles. The use of interactive mixtures enables powders to be handled more easily during manufacture and during filling of the powder into DPI devices. Additionally, the small drug particles are maintained in a relatively dispersed state on the surface of the carrier particles. See e.g. WO 2004/093848 for the preparation of such formulations.

When a DPI is actuated, the fine drug particles should detach from the carrier particles in order that they can be inhaled deep into a patient's respiratory tract. Depending on the nature of the drug substance and the carrier material, the force of adhesion can be strong or weak. If the force of adhesion is too strong, the detachment of fine drug particles from carrier particles can be very low and the resulting fine particle fraction of the dose emitted from a DPI can be very low as to be ineffectual. Powder engineers have developed several means by which they may influence this force of adhesion in order to produce interactive mixtures that possess a quality of adhesion that keeps the interactive mixture together during handling and storage, but which upon actuation of a DPI is not so strong as to prevent the efficient and reproducible re-dispersion of the drug into fine inhalable particles.

One such means is the use of a ternary component in the mixture. Such ternary components are often referred to in the art as force controlling agents or anti-adherent additives. The use of such additives in interactive mixtures is discussed in US 6,521,260. Additives mentioned therein include amino acids, e.g. leucine, isoleucine, lysine, valine, methionine, phenylalanine, and salts of derivatives thereof such as aspartame or acesulfame K; peptides and polypeptides having molecular weight from 0.25 to 1000 KDa, and derivatives thereof; and phospholipids or derivative thereof, e.g. lecithin, more particularly soya lecithin; talc; titanium dioxide; aluminium dioxide; silicon dioxide; and starch.

Other very useful force controlling agents are the salts of fatty acids such as lauric acid, palmitic acid, stearic acid, erucic acid, behenic acid, or derivatives (such as esters and salts) thereof. Specific examples of such materials are: magnesium stearate; sodium stearyl fumarate; sodium stearyl lactylate; phospatidylcholines, phosphatidylglycerols and other examples of natural and synthetic lung surfactants; Liposomal formulations; lauric acid and its salts, for example, sodium lauryl sulphate, magnesium lauryl sulphate; triglycerides such as Dynsan 118 and Cutina HR; and sugar esters in general.

A particularly useful additive has been found to be magnesium stearate. The use of magnesium stearate in interactive mixtures is the subject of US patent 6,645,466.

Interactive mixtures are usually formed by mixing or blending processes. A typical procedure consists of mixing a micronised drug substance with a powdered carrier material. Usually, the carrier material is pre-treated, for example the powder may be preblended in order to change its surface structure and therefore its surface energy in an attempt to manipulate the adhesion forces. When a ternary component is used, it is typical to treat the carrier with it, once again in order to influence the surface energy of the carrier particles.

The benefit of using magnesium stearate in dry powders is taught in US 6,528,096. Specifically it teaches that it can be used to alter the surface properties of carrier particles and thereby improve the properties of dry powder formulations. This reference reports an "advantageous relationship" between surface coating carrier particles with magnesium stearate and the respirable fraction (fine particle fraction) of the emitted dose. Critical to the working of this invention is the need to ensure a continuous coating of magnesium stearate over more than 10% of the surface of the carrier particles. The requisite coating can be achieved by conventional blending of carrier and magnesium stearate, or alternatively mixing techniques applying higher shear forces can be employed. High shear mixing can achieve the requisite coating within about 0.5 hour, however, the skilled person is clearly taught that if conventional blending is to be employed the blending time must be in excess of 2 hours. In The Journal of Aerosol Medicine Vol 11, No. 3, 1998 at 143-152, an inventor of US 6,528,096 teaches that pre-treatment of lactose particles with 0.25% magnesium stearate significantly improves the de-aggregation of beclamethasone dipropionate without causing segregation, i.e. separation of drug and carrier particles, during filling, transport or use.

In Pharmaceutical Research Vol 14, No 11 (Supplement), 1997 at pages S-142-S-143, the authors of whom one is an inventor of US 6,528,096 teach that magnesium stearate was found to be an effective de-stabilizer by the proposed mechanism of reducing the electrostatic attraction force.

It is clear from the prior art that any beneficial properties that derive from the use of magnesium stearate are predicated on it coating carrier particles to some degree and thereby altering the surface properties of the particles. In this regard, the skilled person is taught that in order to advantageously influence the fine particle fraction of an emitted dose, the carrier particles should obtain a continuous or discontinuous surface coating of magnesium stearate. Certain mixing techniques are taught for achieving this result, which involve either high energy mixing, or long duration low energy mixing of magnesium stearates and carrier material. Other suggestions involve combining low energy blending coupled with carrier treatment steps involving high energy milling or mixing. Finally, Wagner et al in Pharmazie 60, 339-344 (2005) in studying the adhesion phenomena of interactive mixtures noted that the energetics of drug-to-carrier interactions are favourable compared to drug-to-drug interactions. This is rationalized on the basis that the fine drug particle sphere has greater contact with the surface of a much larger carrier particle (essentially sphere-to-plate contact) compared to the sphere-sphere contact of adjacent drug particles. Indeed, he reports that in fact, no drug-drug agglomerates were observed in the mixtures studied.

In summary, the prior art recognizes the problem of adhesion in interactive mixtures and suggests that the surface properties of the carrier material should be modified either mechanically or chemically by coating or partially coating with a ternary component. Nowhere is there a suggestion in the art to modify the surface properties of fine drug particles. Indeed, drug-drug interactions appear to be energetically unfavourable relatively speaking and it is perhaps not surprising that the art is concerned with treating carrier particles rather than treating the surface properties of drug particles. However, applicant has found that surface coating carrier particles with a ternary component does not always assure good blend homogeneity in interactive mixtures.

Applicant has now surprisingly found that powder formulations consisting of interactive mixtures of fine drug particles and carrier particles can be obtained if the fine drug particles are pre-treated with a force-controlling agent before blending with carrier particles to form an interactive mixture.

Accordingly, in the present invention there are provided methods of making a powder for inhalation as defined in the appended claims comprising fine drug particles and carrier particles for supporting said drug particles, the formulation further containing a force-controlling agent, wherein the force-controlling agent is disposed on the surface of the fine drug particles as either a particulate coating, or as a continuous or discontinuous film.

The force-controlling agent of the present is magnesium stearate.

The amount of force-controlling agent employed should be at large enough to reduce the auto-adhesion force present between the drug particles. The upper limit depends on the toxicological acceptability of large amounts of force-controlling agent delivered to the lungs. A level of up to 2.0% is preferred. Within these limits, the amount of force-controlling agent employed will depend on the nature of the drug, and the drug loading. The skilled person will have regard the physical and chemical properties of the drug and be able to select an appropriate amount without undue burden or without having to resort to inventive activity.

In a particular embodiment magnesium stearate may be employed in an amount of 0.01 to 2.0% by weight, more particularly 0.1 to 1.5% by weight, still more particularly 0.25 to 1.0% by weight, even more particularly 0.5 to 1.0% by weight. The fine drug particles covered by force-controlling agent particles or a film can be used *per se* since the auto-adhesion forces between drug particles can be largely reduced. However, if the bulk powder properties of any drug/force-controlling agent mixture are inappropriate for use in a dry powder inhaler, the mixture may be further formulated to a suitable powder composition by methods known to the skilled person from the prior art. The resulting powder for inhalation may be in form of a pelletized formulation or of an ordered mixture by adding a carrier material. The carrier material may be any carrier material as specified in the appended claims. As examples thereof, one can mention mono- or di-saccharides such as glucose, glucose mono-hydrate, lactose, lactose mono-hydrate, sucrose or trehalose; sugar alcohols such as mannitol or xylitol; polylactic acid or cyclodextrin; or mixtures thereof. Preferably lactose mono-hydrate is employed.

Any drug substance may be employed in a method according to the present invention. The force-controlling agent is employed in the manner described as means of disrupting inter-particulate forces between drug particles, and promoting the adhesion of drug particles to carrier particles thereby to form the desired interactive mixtures.

There are different types of inter-particulate forces present in bulk powders and their absolute size and relative contribution depend on the surface chemistry and physical characteristics of the particles in the bulk. A general description is given for example in F. Podczeck "Particle-particle Adhesion in Pharmaceutical Powder Handling", Imperial College Press, London 1998, pages 4-15. Hydrophilic interactions are larger than hydrophobic interactions and thus hydrophilic materials are less easy to disperse. This is owing to the capillary force that is generated due to interstitial moisture condensation between contiguous particles, which may be in evidence at ambient air relative humidity values of about 50 %. In addition, porous hydrophilic particles often contain moisture trapped in pores. The capillary force generated by this mechanism is about 10 times stronger than other inter-particulate forces. Between particles of hydrophobic character, capillary forces are less in evidence, which means that strong inter-particulate especially present in hydrophilic compounds are generally not an issue for hydrophobic particles.

Also below a relative humidity value for ambient air of about 50 % hydrophilic particles may show increased inter-particle adhesion because of absorbed moisture acting as a plasticizer. The plasticizing effect results in an increased contact area of the particles and therefore an increased Lifshitz-van der Waals inter-particulate force. From the above it is clear to one skilled in the art that drugs, which consist of soft hydrophilic particles are even more likely to be affected because the true area of contact between the particles may easily increase during storage and normal handling operations of bulk powder.

Hydrophilicity is the tendency of a compound to be solvated by water. More generally, hydrophilic compounds tend to adsorb readily water prior to solvation. The surface chemistry allows such compounds to be wetted readily, and particles of such compounds can form surface water films or layers. Hydrophilic materials are characterized by the fact that they possess a high surface tension value and have the ability to form hydrogen-bonds. There are many parameters that serve to define hydrophilic compounds. One such parameter is the octanol-water partition coefficient. Hydrophilic compounds have low values for this coefficient. In particular, hydrophilic drugs may be characterized for the purpose of the present invention as having a decadic logarithm of the octanol-water partition coefficient (Log P) smaller than 2, more particularly smaller than 1, even more particularly smaller than 0.5.

Another method of characterizing a hydrophilic drug is by measuring its contact angle against water. In particular, hydrophilic drugs for the purpose of the present invention may be characterized by a water contact angle smaller than 90 °, more particularly smaller than 80 °, even more particularly smaller than 70 °.

Notwithstanding the generality of the present invention, having regard to the foregoing, the present invention is particularly advantageous when employed in methods of formulation of hydrophilic drugs.

The following drug substances in particular can be employed in methods of the present invention:
- Active substances may be chosen from beta-mimetics such as Levalbuterol, Terbutalin, Reproterol, Salbutamol, Salmeterol, Formoterol, Fenoterol, Clenbuterol, Bambuterol, Tulobuterol, Broxaterol, Indacaterol, Epinephrin, Isoprenaline or Hexoprenaline; an Anticholinergic such as Tiotropium, Ipratropium, Oxitropium or Glycopyrronium; a Corticosteroid, such as Butixocart, Rofleponide, Budesonide, Ciclesonide, Mometasone, Fluticasone, Beclomethasone, Loteprednol or Triamcinolone; a Leukotrienantagonist, such as Andolast, Iralukast, Pranlukast, Imitrodast, Seratrodast, Zileuton, Zafirlukast or Montelukast; a Phosphodiesterase-Inhibitor, such as Filaminast or Piclamilast; an PAF-Inhibitor, such as Apafant, Forapafant or Israpafant; a potassium channel opener such as Amiloride or Furosemide; a pain killer such as Morphine, Fentanyl, Pentazocine, Buprenorphine, Pethidine, Tilidine, Methadone or Heroin; a potency agent such as Sildenafil, Alprostadil or Phentolamine; or a pharmaceutically acceptable derivative or salt of any of the foregoing compounds or classes of compounds. In as much as any of these compounds possess chiral centres, the compounds can be used in optically pure form, or can be presented as diastereomeric mixtures or racemic mixtures. Methods of the present invention may also employ proteins, peptides, oligopeptides, polypeptides, polyamino acids nucleic acid, polynucleotides, oligo-nucleotides and high molecular weight polysaccharides. Examples of macromolecules that find use in the present invention are:-Albumins (preferably, human serum Insulin; albumin); BSA; IgG; IgM; insulin; GCSF; GMCSF; LHRH; VEGF; hGH; lysozyme; alpha-lactoglobulin; basic fibroblast growth factor basic fibroblast growth factor; (bFGF); asparaginase; tPA; urokinase- VEGF; chymotrypsin; trypsin; streptokinase; interferon; carbonic anhydrase; ovalbumin; glucagon; ACTH; oxytocin; phosphorylase b; alkaline phosphatase- secretin; vasopressin; levothyroxin; phatase; beta-galactosidase; parathyroid hormone, calcitonin; fibrinogen; polyaminoacids (e.g., DNAse, alphal antitrypsin; polylysine, polyarginine); angiogenesis inhibitors or pro- immunoglobulins (e.g., antibodies); moters; somatostatin and analogs; casein; collagen; gelatin; soy protein; and cytokines (e.g., interferon, interleukin); immunoglobulins;
- Physiologically active proteins such as peptide hormones, cytokines, growth factors, factors acting on the cardiovascular system, factors acting on the central and peripheral nervous systems, factors acting on humoral electrolytes and hemal substances, factors acting on bone and skeleton, factors acting on the gastrointestinal system, factors acting on the immune system, factors acting on the respiratory system, factors acting on the genital organs, and enzymes;
- Hormones and hormone modulators including insulin, proinsulin, C-peptide of insulin, a mixture of insulin and C-peptide of insulin, hybrid insulin cocrystals ( Nature Biotechnology, 20, 800-804, 2002 ), growth hormone, parathyroid hormone, luteinizing hormone-releasing hormone (LH-RH), adrenocorticotropic hormone (ACTH), amylin, oxytocin, luteinizing hormone, (D-Tryp6)-LHRH, nafarelin acetate, leuprolide acetate, follicle stimulating hormone, glucagon, prostaglandins, estradiols, testosterone, and other factors acting on the genital organs and their derivatives, analogues and congeners. As analogues of said LH-RH, such known substances as those described in U.S. Pat. Nos. 4,008,209, 4,086,219, 4,124,577, 4,317,815 and 5,110,904 can be mentioned;
- Hematopoietic or thrombopoietic factors include, among others, erythropoietin, granulocyte colony stimulating factor (G-CSF), granulocyte-macrophage stimulating factor (GM-CSF) and macrophage colony stimulating factor (M-CSF), leukocyte proliferation factor preparation (Leucoprol, Morinaga Milk), thrombopoietin, platelet proliferation stimulating factor, megakaryocyte proliferation (stimulating) factor, and factor VIII;
- Therapeutic factors acting on bone and skeleton and agents for treating osteoporosis including bone GLa peptide, parathyroid hormone and its active fragments ( osteostatin, Endocrinology 129, 324, 1991 ), histone H4-related bone formation and proliferation peptide (OGP, The EMBO Journal 11, 1867, 1992 ) and their muteins, derivatives and analogs thereof;
- Enzymes and enzyme cofactors including pancrease, L-asparaginase, hyaluronidase, chymotrypsin, trypsin, tPA, streptokinase, urokinase, pancreatin, collagenase, trypsinogen, chymotrypsinogen, plasminogen, streptokinase, adenyl cyclase, and superoxide dismutase (SOD);
- Vaccines include Hepatitis B, MMR (measles, mumps, and rubella), and Polio vaccines;
- Growth factors include nerve growth factors (NGF, NGF-2/NT-3), epidermal growth factor (EGF), fibroblast growth factor (FGF), insulin-like growth factor (IGF), transforming growth factor (TGF), platelet-derived cell growth factor (PDGF), and hepatocyte growth factor (HGF);
- Factors acting on the cardiovascular system including factors which control blood pressure, arteriosclerosis, etc., such as endothelins, endothelin inhibitors, endothelin antagonists described in EP 436189, 457195 , 496452 and 528312, JP [Laid Open] No. H-3-94692/1991 and 130299/1991, endothelin producing enzyme inhibitors vasopressin, renin, angiotensin I, angiotensin II, angiotensin III, angiotensin I inhibitor, angiotensin II receptor antagonist, atrial naturiuretic peptide (ANP), and antiarrythmic peptide ; Factors acting on the central and peripheral nervous systems including opioid peptides (e.g. enkephalins, endorphins), neurotropic factor (NTF), calcitonin gene-related peptide (CGRP), thyroid hormone releasing hormone (TRH), salts and derivatives of TRH [ JP [Laid Open]No. 50-121273/1975 ( U.S. Pat. No. 3,959,247 ), JP [Laid Open]No. 52-116465/1977 ( U.S. Pat. No. 4,100,152 )], and neurotensin;
- Factors acting on the gastrointestinal system including secretin and gastrin;
- Factors acting on humoral electrolytes and hemal substances including factors which control hemagglutination, plasma cholesterol level or metal ion concentrations, such as calcitonin, apoprotein E and hirudin. Laminin and intercellular adhesion molecule 1 (ICAM 1) represent exemplary cell adhesion factors;
- Factors acting on the kidney and urinary tract including substances which regulate the function of the kidney, such as brain-derived natriuretic peptide (BNP), and urotensin;
- Factors which act on the sense organs including factors which control the sensitivity of the various organs, such as substance P;
- Chemotherapeutic agents, such as paclitaxel, mytomycin C, BCNU, and doxorubicin;
- Factors acting on the immune system including factors which control inflammation and malignant neoplasms and factors which attack infective microorganisms, such as chemotactic peptides and bradykinins; and
- Naturally occurring, chemically synthesized or recombinant peptides or proteins which may act as antigens, such as cedar pollen and ragweed pollen, and these materials alone or together with coupled to haptens, or together with an adjuvant.

The present invention is particularly useful in methods of formulation of hydrophilic and moisture sensitive active substances, such as the salt forms of any of the compounds mentioned above such as the chloride, bromide, iodide, nitrate, carbonate, sulphate, methylsulphate, phosphate, acetate, benzoate, benzensulphonate, fumarate, malonate, tartrate, succinate, citrate, lactate, gluconate, glutamate, edentate, mesylate, pamoate, pantothenate or hydroxynaphthoate; or an ester form such as an acetate, propionate, phosphate, succinate or etabonate.

Methods for making formulations containing a beta-mimetic, an anti-cholinergic or a corticosteroid, alone or in any combination thereof constitute preferred embodiments of the present invention. These actives may be present in salt or ester form, such as a beta-mimetic in salt form, e.g. levalbuterol sulphate, formoterol fumarate, formoterol tartrate, salbutamol sulphate or salmeterol xinafoate (salmeterol 1-hydroxy-2-naphthoate); or a corticosteroid in the form of an ester, such as beclamethasone dipropionate, fluticasone propionate, triamcinoline 16,21-diacetate, triamcinoline acetonide 21-acetate, triamcinoline acetonide 21-disodium phosphate, triamcinoline acetonide 21-hemisuccinate, mometasone furoate, or loteprednol etabonate.

In a most preferred method of the present invention the formulation contains an anti-cholinergic agent in salt form such as oxitropium bromide, glycopyrronium bromide (glycopyrrolate), ipratropium bromide or tiotropium bromide.

The powder may suitably be administered to parenterally to an human patient, in particular by inhalation, using, for example, a DPI.

The formulations prepared by the methods of the present invention may be prepared in such a manner that the drug is first brought in contact with the appropriate amount of force-controlling agent. Close contact of particles of force-controlling agent with drug particles is necessary and important to achieve a reduction of the auto-adhesion forces established in the bulk drug powder. The close contact can be achieved by methods known to the skilled person. Particularly, screening both drug and agent through a narrow sieve gives a neat dispersion of both particle populations. Appropriately sized sieves for this operation are e.g. 25 to 250 micrometer size (500 to 60 mesh according to BS 410), more particularly 25 to 180 micrometer (500 to 85 mesh according to BS 410), even more particularly 25 to 90 micrometer (500 to 170 mesh according to BS 410).

Additionally or alternatively, blending and mixing apparatus may be applied to achieve close inter-particle contact, for example tumble blenders, bin blenders, conical blenders and the like. Particularly, tumble blending may be applied for this purpose.

If the fine drug particles to be formulated as a powder formulation for inhalation are not of the appropriate size suitable for the inhaled route, particle reduction techniques in the presence of force-controlling agent can be applied to yield particles of suitable size. The preferred method is co-micronisation using an air jet mill which again brings the drug particles and the particles of force-controlling agent in such a contact that either a continuous or discontinuous film is formed or the agent particles adhere to the drug particle surface. However, any technique known in the art and suitable for co-micronisation can be employed.

The blending step described above is preferably carried out as one of a series of blending steps described below.

In a first step, one or more drugs and force-controlling agent are mixed together in such a way that the agent adheres to the surface of the drug particles either as a particulate coating or as a continuous or discontinuous film. As stated hereinabove, the treated drug particles may possess appropriate properties that enable them to be used alone in a dry powder inhaler device. However, if desired they may be further mixed with a carrier material.

Accordingly, the treated fine drug particles are mixed with a carrier material. This mixing step is preferably carried out in a powder blender for a period not exceeding one hour and preferably less than 30 minutes, more particularly less than 20 minutes, for example about 15 to 20 minutes.

The carrier material may be used untreated or it may be treated in the same manner as the fine drug particles.

In the methods of the present invention, the drug, force-controlling agent and carrier material can be as stated already herein above.

If the amount of drug used in the formulation is low, e.g. less than about 30% by weight of the formulation, more particularly about 1 % to 20 % by weight, even more particularly about 0.01 to 10% by weight of the formulation, it is preferred that after the treatment of the fine drug particles, the resulting drug/force-controlling agent mixture is blended with a small portion of the carrier material, e.g. about 10%, to form a powder mixture relatively concentrated with respect to the drug. This is to ensure adequate mixing of the drug with the carrier material. Subsequently, an additional step is employed to mix the remaining carrier material with the concentrated mixture from the earlier step. Again, this is preferably carried out in a powder blender. It is preferred that no other blending step is carried out. However, it may be deemed necessary to perform additional blending and sieving steps to achieve a final powder formulation of suitable quality.

To ensure the powder ingredients are of the appropriate particle size it is customary to prepare the ingredients by screening through appropriate sized sieves, e.g. 25 to 500 micrometer size (500 to 30 mesh according to BS 410), more particularly 63 to 250 micrometer (240 to 60 mesh according to BS 410).

In order that the fine drug particles are inhalable, i.e. in order that they can pass into the deep lung such as the terminal and respiratory bronchioles and the alveolar ducts and sacs, they must be in particulate form having a mean particle diameter (measured as the mass mean aerodynamic diameter) of at most about 10 micrometers, e.g. from 1 to 10 micrometers, and preferably 1 to 6 micrometers, even more preferably 1 to 4 micrometers. Such micro-fine particles can be obtained in a manner known *per se,* for example by micronisation, controlled precipitation from selected solvents, or by spray drying.

The amount of drug employed may vary within wide limits depending on the nature of the drug, the type and severity of the condition to be treated and the condition of the patient in need of treatment.

For drugs employed to treat local conditions of the lung such as all manner of asthma and chronic obstructive pulmonary disease, relatively low doses of drug can be employed, for example about 5 to 5000 micrograms, more particularly 5 to 500 micrograms. For drugs that are intended to be delivered systemically through the lung, one may need higher doses to take into account issues relating to absorption through the lung and into the blood plasma. Typically, one might employ drugs at levels of about 20 micrograms to 50 milligrams, more particularly 50 micrograms to 20 milligrams.

Expressed as a concentration based on the total weight of the formulation, the drug may be present in amounts of 0.01 to 30% by weight, more particularly 0.1 to 10% by weight, more particularly 0.1 to 5% by weight. It is not surprising therefore that to achieve dosage accuracy, the drug must be diluted with carrier material. In a typical formulation the carrier material may be present in amounts of up to 99% by weight or more, in particular 50 to 99% by weight, depending on the particular dilution desired and on the amount of force-controlling agent employed in the formulation. The dilution is chosen such that an acceptable shot weight delivered from an inhaler contains exactly the desired dose of drug. In this regard, the exact dose may be delivered in a single shot or multiple shots. Dilution is also used to affect powder mixtures having good macroscopic properties such as flowability, and to balance adhesive or cohesive forces of the micro-fine active substance to ensure good homogeneity of the formulation.

Nucleic acids, including double-stranded or single-stranded polynucleotide, oligonucleotide or short nucleic acid sequences may also be formulated according to the present invention. The term nucleic acid includes both RNA (e.g. siRNA, mRNA, ribozymes, aptamers) and DNA (e.g. cDNA or genomic DNA). The nucleic acid may be present in the form of a vector (e.g. a plasmid or other construct) with suitable sequences to direct or control expression (i.e. a promoter sequence).

Carrier materials employed must be in the form of sufficiently large particle size such that they can be easily handled during manufacture and filling operations. They should also be large enough such that they are not inhalable into the deep lung. Typically a carrier material will have a mean particle diameter (measured as the mass mean aerodynamic diameter) of about 10 to 500 micrometers, and preferably 50 to 500 or 50 to 300 micrometers.

Dry powder formulations made according to the present invention are particularly suitable for use in multi-dose dry powder inhalers. In particular, the formulations are suitable for use in such inhalers, which comprise a reservoir from which individual therapeutic dosages can be withdrawn on demand through actuation of the device. However, the formulations are also useful in multi-dose inhalers that contain a plurality of capsules containing single or multiple pre-dosed units.

Typical of such multi-dose inhaler device suitable for use with formulations made according to the present invention is described in US 6182655.

Multi-dose inhalers may contain a reservoir of dry powder that contains tens or even hundreds of therapeutic doses. The term "therapeutic dose(s)" as used herein means an amount of inhalation formulation containing a requisite amount of drug to illicit a therapeutic effect, e.g. to alleviate, prevent or inhibit the particular condition to be treated, when delivered to a patient. A therapeutic dose may be delivered with one or more actuations of a DPI device. This is because the amount of powderthat can be delivered to a patient without irritating the patient, e.g. making the patient cough, or what can reasonably or comfortably be delivered within a single inspiration, is limited to about 50mg per actuation, more particularly 25mg per actuation. Accordingly, depending on the nature of the drug and the nature and severity of the condition to be treated, one or more actuations may be necessary per number of hours, per day, for any number of days, weeks, months and so-forth.

The therapeutic dose will depend largely on the nature of the drug, the condition of the patient, and the nature and severity of the condition to be treated. A therapeutic dose may range between as little as 1 ng/kg, for example when treating a local condition such as asthma with a potent active substance to as much as 10mg/kg, more particularly dose will range from 20ng/kg to 1 mg/kg. The therapeutic dose will be indicated on packaging or labelling accompanying the DPI device and is specifically referred to in the Label Claim.

In order to ensure inter-batch quality and reproducibility, formulations should be tested in order to ensure that the mean dose of formulation emitted from a MDI, should not vary considerably from the Label Claim. In this regard, the formulations made according to the present invention are particularly stable, for example they meet the following standards:
- The Mean Delivered Dose is within +/- 15% of the Label Claim, and 9 from 10 at least of single doses are not outside +/- 25% of the mean, and all single doses are within +/- 35% of the mean; or
- At least 9 from 10 single doses are within +/- 20% of the Label Claim, and all single doses are within +/-25% of the Label Claim.

The Shot Weight and Delivered Dose and their variance can be measured using the Dosage Unit Sampling Apparatus (DUSA). The fine particle fraction (FPF) can be measured using an Andersen Cascade Impactor (ACI). The measurement methodology and the apparatus therefore are well known in the art, and are described in the United States Pharmacopoeia Chapter <601>, or in the inhalants monograph of the European Pharmacopoeia, both of which documents are hereby incorporated by reference. The USP states that the Apparatus 1 should be used for the measurement of FPF. The USP also states that Delivered Dose Uniformity should be measured with DUSA or its equivalent. However, the Delivered Dose and Delivered Dose uniformity are preferably measured using the so-called Funnel Method. The Funnel Method is described in Drug Delivery to the Lungs, VIII p116 to 119, which is hereby incorporated by reference. In summary, the Funnel Method consists of discharging a formulation from a DPI into a Funnel Apparatus, which basically consists of a standard Buchner Funnel. The discharged dose is captured on the glass sinter of the Funnel, and can be washed off, and the dose determined using HPLC analysis. The Funnel Method gives comparable results to the standard USP apparatus, and is generally considered to be an equivalent of the DUSA apparatus. Fine particle fraction measured according to the above described methodology is considered to consist of the combined fractions collected from stages 2 to Filter Stage of an Andersen Cascade Impactor calibrated at 60 L/min air flow rate. These fractions have an aerodynamic particle size of less than 3.2 micrometers.

Alternatively, Fine Particle Fraction can be measured by the Twin Impinger Method and the Multi-stage Liquid Impinger Method as are described in the Pharmacopoeia, and as are set forth in the Examples below.

Formulations made according to the present invention meet pharmacopoeia requirements as to Delivered Dose Uniformity as set forth, for example in the United States and European Pharmacopoeias. For example, the formulations meet the requirement set out in the USP26-NF21 chapter <601> "Delivered Dose Uniformity". Indeed, the formulations appear to be so stable that they may even meet the relatively more stringent Delivered Dose Uniformity requirements set forth in the current Draft Guidance from the FDA, published by the CDER in October 1998. Still further, the Delivered Dose of the formulations contains a high fraction of fine particles, i.e. particles that are capable of penetrating the deep lung, e.g. having a diameter of less than about 4.7 micrometers, as measured by the ACI; below 6.4 as measured by the Twin Impinger; and below 6.8 as measured by the Multi-stage Liquid Impinger.

There now follows a series of examples that serve to illustrate the invention.

### Method Particle Fraction Size Measurement Method

Assemble the Andersen Cascade Impactor according to manufacturer's instructions with a suitable filter in place and ensure that the system is airtight. To ensure efficient particle capture, coat each plate with a high viscosity liquid deposited from a volatile solvent. The pre-separator should be coated in the same way or should contain 10 ml of a suitable solvent. Connect the apparatus to a flow system comprising flow control valve, two-way valve, timer and vacuum pump.

The test is conducted at a flow rate adapted to the internal resistance of the inhaler device drawing 4 litres of air through the apparatus. At high flow rates it may be necessary to remove the lowest stages from the stack. For adjustment of the flow rate connect a flow meter, calibrated for the volumetric flow leaving the meter, to the induction port. Adjust the flow control valve to achieve steady flow through the system at the required rate.

Ensure that critical flow occurs in the flow control valve by measuring the absolute pressure on both sides of the flow control valve. Switch off the airflow.

Prepare the dry-powder inhaler for use according to the patient instructions. With the pump running and the two-way valve closed, locate the mouthpiece of the inhaler in the mouthpiece adapter. Discharge the powder into the apparatus by opening the valve for the time required for drawing 4 litres of air through. Repeat the discharge sequence. The number of discharges should be minimised and typically would not be greater than ten. The number of discharges should be sufficient to ensure an accurate and precise determination of fine particle dose. After the final discharge, wait for 5 seconds and then switch off the pump.

Dismantle the apparatus. Carefully remove the filter and extract the active ingredient into an aliquot of the solvent. Remove the pre-separator, induction port and mouthpiece adapter from the apparatus and extract the drug into an aliquot of the solvent. Extract the active ingredient from the inner walls and the collection plate of each of the stages of the apparatus into aliquots of solvent. Using a suitable method of analysis, determine the quantity of drug contained in each of the nine volumes of solvent.

Calculate the mass of drug deposited on each stage per discharge and the mass of drug per discharge deposited in the induction port, mouthpiece adapter and where used the pre-separator. The total mass of the drug is not less than 75 per cent and not more than 125 per cent of the average delivered dose determined during testing for uniformity of delivered dose. If the total mass is outside this range the test must be repeated.
Starting at the filter, derive a cumulative mass vs. cut-off diameter of the respective stages. Calculate the Fine Particle Dose (FPD) by interpolation the mass of drug less than 5 µm. If necessary, and where appropriate, plot the cumulative fraction of drug versus cut-off diameter on log probability paper, and use this plot to determine values for the Mass Median Aerodynamic Diameter (MMAD) and the Geometric Standard Deviation (GSD).

### Example 1

### Formulation 1

A powder formulation consisting of glycopyrrolate (glycopyrronium bromide), magnesium stearate and lactose monohydrate is formed as follows:Glycopyrrolate and magnesium stearate are screened through a 38 micrometer sieve. Lactose monohydrate is screened through a 250 micrometer sieve. The sieved glycopyrrolate-magnesium stearate bulk powder is mixed with about half the amount of sieved lactose monohydrate in a Turbula T2C powder blender at 22 rpm for 10 minutes.

The resulting concentrated mixture is sieved through a 250 micrometer sieve, the remaining lactose monohydrate is added and the mixture blended for a further 10 minutes at 22 rpm in the blender.

The dry powder blend achieved is homogeneous when assessed visually and under the microscope. The blend has satisfying blend homogeneity with a relative standard deviation of the drug content of the withdrawn samples below 5 %, usually even below 3 %.

### Formulation 2 (for comparison, not part of the invention)

A dry powder formulation consisting of glycopyrrolate (glycopyrronium bromide), magnesium stearate and lactose monohydrate is formed according to a prior art process as follows:Lactose monohydrate and magnesium stearate are screened through a 250 micrometer sieve and mixed in a Turbula T2C powder blender at 30 rpm for 20 minutes.

Glycopyrrolate and about half of the lactose-magnesium stearate mixture are screened through a 250 micrometer sieve and mixed in a Turbula T2C powder blender at 46 rpm for 20 minutes.

Eventually, the resulting concentrated mixture and the remaining lactose monohydrate are sieved through a 250 micrometer sieve and the mixture blended for a 10 minutes at 46 rpm in the blender.

The dry powder blend achieved has an inhomogeneous aspect when assessed visually and under the microscope. The blend homogeneity test gives a relative standard deviation of the drug content of the withdrawn samples of more than 5 %.

### Example 2

### Formulation 3 (not part of the invention)

A dry powder formulation consisting of glycopyrrolate (glycopyrronium bromide), magnesium stearate and lactose monohydrate is formed according to the following method:Glycopyrrolate and magnesium stearate are screened through a 38 micrometer sieve. Lactose monohydrate is screened through a 250 micrometer sieve. Both sieved bulk powders are mixed in a high shear mixer Niro PP1 for 10 minutes at 300 rpm impeller speed and 300 rpm chopper speed.

The powder blend achieved is homogeneous when assessed visually and under the microscope. The blend has satisfying blend homogeneity with a relative standard deviation of the drug content of the withdrawn samples below 5 %, usually even below 3 %.

### Example 3 : Measurement of Fine Particle Fraction

The formulations 1 and 2 employed are those formed according to Example 1 above. The powder blends thus produced are filled into SkyePharma proprietary dry powder inhalers Skyehaler^{™} as more fully described in US patent 6,182,655 for assessment of Dose Content Uniformity and fine particle fraction of the delivered dose.

After filling the formulations in the DPI devices, the devices are allowed to stand for at least 24 hours before testing.

The aerodynamic particle size distribution is determined using the Andersen Cascade Impactor Mark II, equipped with pre-separator and 8 stages, designed and calibrated for 60L/min flow rate (apparatus D of the Eur. Pharmacopoeia 4.4 section 2.9.18). The fine particle dose is the amount of drug that is found on the stages 2 to the filter stage of this apparatus.

3 actuations of the formulations of Example 1 and 2 are discharged into the particle sizing apparatus specified above by pulling 4 L of air through the apparatus at a set flow rate of 60 L/min. Delivered and aerosolised drug particles are classified in accordance with their particle momentum achieved in the flow which depends on the equivalent aerodynamic particle size. Thus fractions of the dose are deposited at different parts or collecting stages of the apparatus, in accordance with the aerodynamic particle size of the drug particles. Each fraction is collected, adjusted to volume and analysed using HPLC.

HPLC analysis of Formulation 1 showed that the fine particle fraction (less than 3.2 micrometers) of the dose delivered into the Andersen Cascade Impactor apparatus is about 42 %.

HPLC analysis of Formulation 3 showed that the fine particle fraction (less than 3.2 micrometers) of the dose delivered into the Andersen Cascade Impactor apparatus is about 43 %.

## Claims

1. A method of making a powder for inhalation comprising:
a) a first step of sieving a force-controlling agent with particles of one or more pharmacologically active materials in order to obtain a mixture wherein the particles of said force-controlling agent are disposed on the surface of the active particles as either a particulate coating, or as a continuous or discontinuous film; and then
b) a second step of mixing or blending said mixture with a carrier material;
**characterised in that** the mixing or blending in step b) is carried out in a diffusion blender, tumble blender, bin blender or conical blender, the sieving is carried out in a sieve having a size of 25 to 250 micrometres; the force-controlling agent is magnesium stearate; and the carrier material is selected from mono- or di-saccharides such as glucose, lactose, lactose mono-hydrate, sucrose or trehalose; sugar alcohols such as mannitol or xylitol; polylactic acid; or mixtures thereof and has a mean particle diameter of 50 to 500 micrometres measured as the mass mean aerodynamic diameter.

2. A method according to claim 1 wherein in b) in a first step the mixture is mixed or blended with a first portion of the carrier material to form a second mixture, and in a subsequent step the remainder of the carrier material is mixed with said second mixture.

3. A method of making a powder for inhalation comprising:
a) a first step of mixing, blending or sieving a force-controlling agent with particles of one or more pharmacologically active materials in order to obtain a mixture wherein the particles of said force-controlling agent are disposed on the surface of the active particles as either a particulate coating, or as a continuous or discontinuous film; and then
b) a second step of mixing or blending said mixture with a carrier material;
**characterised in that** the mixing or blending in steps a) and b) is carried out in a diffusion blender, tumble blender, bin blender or conical blender, the sieving is carried out in a sieve having a size of 25 to 250 micrometres; the force-controlling agent is magnesium stearate; and the carrier material is selected from mono- or di-saccharides such as glucose, lactose, lactose mono-hydrate, sucrose or trehalose; sugar alcohols such as mannitol or xylitol; polylactic acid; or mixtures thereof and has a mean particle diameter of 50 to 500 micrometres measured as the mass mean aerodynamic diameter
wherein in b) in a first step the mixture is mixed or blended with a first portion of the carrier material to form a second mixture, and in a subsequent step the remainder of the carrier material is mixed with said second mixture.

4. A method according to any one of the preceding claims wherein the force-controlling agent is present in amounts of up to 5.0% by weight.

5. A method according to any one of the preceding claims wherein the force-controlling agent is present in amounts of 0.01 to 5.0% by weight.

6. A method according to any one of the preceding claims wherein the carrier material is lactose mono-hydrate.

7. A method according to any one of the preceding claims wherein the drug is selected from those drugs having a contact angle against water that is less than 90°.

8. A method according to any one of claims 1 to 7 wherein the drug is selected from those drugs having an octanol-water partition coefficient (log P) smaller than 2.

9. A method according to any one of the claims 1 to 8 wherein the drug is selected from the group consisting of a beta-mimetic selected from the group consisting of Levalbuterol, Terbutalin, Reproterol, Salbutamol, Salmeterol, Formoterol, Fenoterol, Clenbuterol, Bambuterol, Tulobuterol, Broxaterol, Epinephrin, Isoprenaline or Hexoprenaline; an anticholinergic selected from the group consisting of Tiotropium, Ipratropium, Oxitropium or Glycopyrronium; a Corticosteroid selected from the group consisting of Butixocart, Rofleponide, Budesonide, Ciclosenide, Mometasone, Fluticasone, Beclomethasone, Loteprednol or Triamcinolone;a Leukotriene antagonist selected from the group consisting of Andolast, Iralukast, Pranlukast, Imitrodast, Seratrodast, Zileuton, Zafirlukast or Montelukast;a Phosphodiesterase-Inhibitor selected from the group consisting of Filaminast or Piclamilast; aPAF-Inhibitor selected from the group consisting of Apafant, Forapafant or Israpafant; a potassium channel opener selected from the group consisting of Amiloride or Furosemide; a pain killer selected from the group consisting of Morphine, Fentanyl, Pentazocine, Buprenorphine, Pethidine, Tilidine, Methadone or Heroin; a potency agent selected from the group consisting of Sildenafil, Alprostadil or Phentolamine; proteins, peptides, oligopeptides, polypeptides, polyamino acids, nucleic acids, polynucleotides, oligo-nucleotides and high molecular weight polysaccharides; macromolecules, selected from the group consisting of: Albumins (preferably, human serum Insulin; albumin); BSA; IgG; IgM; insulin; GCSF; GMCSF; LHRH; VEGF; hGH; lysozyme; alpha-lactoglobulin; basic fibroblast growth factor; (bFGF); asparaginase; tPA; urokinase- VEGF; chymotrypsin; trypsin; streptokinase; interferon; carbonic anhydrase; ovalbumin; glucagon; ACTH; oxytocin; phosphorylase b; alkaline phosphatase- secretin; vasopressin; levothyroxin; phosphatase; beta-galactosidase; parathyroid hormone, calcitonin; fibrinogen; polyaminoacids selected from DNAse, alphal antitrypsin; polylysine, polyarginine; angiogenesis inhibitors or pro-immunoglobulins; promoters; somatostatin and analogs; casein; collagen; gelatin; soy protein; and cytokines; immunoglobulins; peptide hormones, cytokines, growth factors, factors acting on the cardiovascular system, factors acting on the central and peripheral nervous systems, factors acting on humoral electrolytes and hemal substances, factors acting on bone and skeleton, factors acting on the gastrointestinal system, factors acting on the immune system, factors acting on the respiratory system, factors acting on the genital organs, and enzymes; hormones and hormone modulators including insulin, proinsulin, C-peptide of insulin, a mixture of insulin and C-peptide of insulin, hybrid insulin cocrystals; growth hormone, parathyroid hormone, luteinizing hormone-releasing hormone (LH-RH), adrenocorticotropic hormone (ACTH), amylin, oxytocin, luteinizing hormone, (D-Tryp6)-LHRH, nafarelin acetate, leuprolide acetate, follicle stimulating hormone, glucagon, prostaglandins, estradiols, testosterone, and other factors acting on the genital organs and their derivatives, analogues and congeners; hematopoietic or thrombopoietic factors selected from the group consisting of erythropoitin, granulocyte colony stimulating factor (G-CSF), granulocyte-macrophage stimulating factor (GM-CSF) and macrophage colony stimulating factor (M-CSF), leukocyte proliferation factor preparation (Leucoprol, Morinaga Milk), thrombopoietin, platelet proliferation stimulating factor, megakaryocyte proliferation (stimulating) factor, and factor VIII; therapeutic factors acting on bone and skeleton and agents for treating osteoporosis including bone GLa peptide, parathyroid hormone and its active fragments, histone H4-related bone formation and proliferation peptide and their muteins, derivatives and analogs thereof; enzymes and enzyme cofactors selected from the group consisting of pancrease, L-asparaginase, hyaluronidase, chymotrypsin, trypsin, tPA, streptokinase, urokinase, pancreatin, collagenase, trypsinogen, chymotrypsinogen, plasminogen, streptokinase, adenyl cyclase, and superoxide dismutase (SOD); vaccines include Hepatitis B, MMR (measles, mumps, and rubella), and Polio vaccines; growth factors include nerve growth factors (NGF, NGF-2/NT-3), epidermal growth factor (EGF), fibroblast growth factor (FGF), insulin-like growth factor (IGF), transforming growth factor (TGF), platelet-derived cell growth factor (PDGF), and hepatocyte growth factor (HGF); factors acting on the cardiovascular system including factors which control blood pressure and arteriosclerosis, selected from endothelins, endothelin inhibitors and endothelin antagonists; endothelin producing enzyme inhibitors vasopressin, renin, angiotensin I, angiotensin II, angiotensin III, angiotensin I inhibitor, angiotensin II receptor antagonist, atrial natriuretic peptide (ANP), and antiarrythmic peptide; Factors acting on the central and peripheral nervous systems including opioid peptides (e.g. enkephalins, endorphins), neurotropic factor (NTF), calcitonin gene-related peptide (CGRP), thyroid hormone releasing hormone (TRH), salts and derivatives of TRH, and neurotensin; factors acting on the gastrointestinal system including secretin and gastrin; factors acting on humoral electrolytes and hemal substances including factors which control hemagglutination, plasma cholesterol level or metal ion concentrations, such as calcitonin, apoprotein E and hirudin; Laminin and intercellular adhesion molecule 1 (ICAM 1) represent exemplary cell adhesion factors; factors acting on the kidney and urinary tract including substances which regulate the function of the kidney, such as brain-derived natriuretic peptide (BNP), and urotensin; factors which act on the sense organs including factors which control the sensitivity of the various organs; chemotherapeutic agents, selected from the group consisting of paclitaxel, mitomycin C, BCNU, and doxorubicin; factors acting on the immune system including factors which control inflammation and malignant neoplasms and factors which attack infective microorganisms, selected from the group consisting of chemotactic peptides and bradykinins; and naturally occurring, chemically synthesized or recombinant peptides or proteins which may act as antigens, such as cedar pollen and ragweed pollen, and these materials alone or together coupled to haptens, or together with an adjuvant; and a pharmaceutically acceptable derivative or salt of any of the foregoing compounds or classes of compounds.

## Patentansprüche

1. Verfahren zur Herstellung eines Pulvers zur Inhalation, umfassend:
(a) einen ersten Schritt zum Sieben eines kraftkontrollierenden Mittels mit Partikeln von einem oder mehr pharmakologisch aktiven Material(ien) zum Erhalt eines Gemischs, wobei die Partikel des genannten kraftkontrollierenden Mittels auf der Oberfläche der aktiven Partikel entweder als ein partikulärer Überzug oder als ein kontinuierlicher oder diskontinuierlicher Film angeordnet sind; und dann
(b) einen zweiten Schritt zum Mischen oder innigen Vermischen des genannten Gemischs mit einem Trägermaterial;
**dadurch gekennzeichnet, dass** das Mischen oder innige Vermischen in Schritt b) in einem Diffusionsmischer, einem Taumelmischer, einem Behältermischer oder einem Konusmischer durchgeführt wird, das Sieben in einem Sieb mit einer Größe von 25 µm bis 250 µm durchgeführt wird; das kraftkontrollierende Mittel Magnesiumstearat ist; und das Trägermaterial ausgewählt ist aus Mono- oder Disacchariden, wie zum Beispiel Glucose, Lactose, Lactose-Monohydrat, Saccharose oder Trehalose; Zuckeralkoholen, wie zum Beispiel Mannitol oder Xylitol; Polymilchsäure; oder Gemischen davon und einen als massenmittleren aerodynamischen Durchmesser gemessenen mittleren Partikeldurchmesser von 50 µm bis 500 µm aufweist.

2. Verfahren nach Anspruch 1, wobei in b) in einem ersten Schritt das Gemisch mit einem ersten Anteil des Trägermaterials zur Bildung eines zweiten Gemischs gemischt oder innig vermischt wird, und in einem sich anschließenden Schritt der Rest des Trägermaterials mit dem genannten zweiten Gemisch gemischt wird.

3. Verfahren zur Herstellung eines Pulvers zur Inhalation, umfassend:
(a) einen ersten Schritt zum Mischen, innigen Vermischen oder Sieben eines kraftkontrollierenden Mittels mit Partikeln von einem oder mehr pharmakologisch aktiven Material(ien) zum Erhalt eines Gemischs, wobei die Partikel des genannten kraftkontrollierenden Mittels auf der Oberfläche der aktiven Partikel entweder als ein partikulärer Überzug oder als ein kontinuierlicher oder diskontinuierlicher Film angeordnet sind; und dann
(b) einen zweiten Schritt zum Mischen oder innigen Vermischen des genannten Gemischs mit einem Trägermaterial;
**dadurch gekennzeichnet, dass** das Mischen oder innige Vermischen in den Schritten a) und b) in einem Diffusionsmischer, einem Taumelmischer, einem Behältermischer oder einem Konusmischer durchgeführt wird, das Sieben in einem Sieb mit einer Größe von 25 µm bis 250 µm durchgeführt wird; das kraftkontrollierende Mittel Magnesiumstearat ist; und das Trägermaterial ausgewählt ist aus Mono- oder Disacchariden, wie zum Beispiel Glucose, Lactose, Lactose-Monohydrat, Saccharose oder Trehalose; Zuckeralkoholen, wie zum Beispiel Mannitol oder Xylitol; Polymilchsäure; oder Gemischen davon und einen als massenmittleren aerodynamischen Durchmesser gemessenen mittleren Partikeldurchmesser von 50 µm bis 500 µm aufweist,
wobei in b) in einem ersten Schritt das Gemisch mit einem ersten Anteil des Trägermaterials zur Bildung eines zweiten Gemischs gemischt oder innig vermischt wird, und in einem sich anschließenden Schritt der Rest des Trägermaterials mit dem genannten zweiten Gemisch gemischt wird.

4. Verfahren nach einem der vorangehenden Ansprüche, wobei das kraftkontrollierende Mittel in Mengen bis zu 5,0 Gew.-% vorliegt.

5. Verfahren nach einem der vorangehenden Ansprüche, wobei das kraftkontrollierende Mittel in Mengen von 0,01 Gew.-% bis 5,0 Gew.-% vorliegt.

6. Verfahren nach einem der vorangehenden Ansprüche, wobei das Trägermaterial Lactose-Monohydrat ist.

7. Verfahren nach einem der vorangehenden Ansprüche, wobei das Arzneimittel aus den Arzneimitteln ausgewählt ist, die einen Kontaktwinkel gegen Wasser aufweisen, der kleiner als 90° ist.

8. Verfahren nach einem der Ansprüche 1 bis 7, wobei das Arzneimittel aus den Arzneimitteln ausgewählt ist, die einen Octanol-Wasser-Verteilungskoeffizienten (log P) von kleiner als 2 aufweisen.

9. Verfahren nach einem der Ansprüche 1 bis 8, wobei das Arzneimittel aus der Gruppe ausgewählt ist, bestehend aus einem Beta-Mimetikum, das aus der Gruppe ausgewählt ist, bestehend aus Levalbuterol, Terbutalin, Reproterol, Salbutamol, Salmeterol, Formoterol, Fenoterol, Clenbuterol, Bambuterol, Tulobuterol, Broxaterol, Epinephrin, Isoprenalin oder Hexoprenalin; einem Anticholinergikum, das aus der Gruppe ausgewählt ist, bestehend aus Tiotropium, Ipratropium, Oxitropium oder Glycopyrronium; einem Corticosteroid, das aus der Gruppe ausgewählt ist, bestehend aus Butixocart, Rofleponid, Budesonid, Ciclosenid, Mometason, Fluticason, Beclomethason, Loteprednol oder Triamcinolon; einem Leukotrienantagonisten, der aus der Gruppe ausgewählt ist, bestehend aus Andolast, Iralukast, Pranlukast, Imitrodast, Seratrodast, Zileuton, Zafirlukast oder Montelukast; einem Phosphodiesterase-Hemmer, der aus der Gruppe ausgewählt ist, bestehend aus Filaminast oder Piclamilast; einem PAF-Hemmer, der aus der Gruppe ausgewählt ist, bestehend aus Apafant, Forapafant oder Israpafant; einem Kaliumkanalöffner, der aus der Gruppe ausgewählt ist, bestehend aus Amilorid oder Furosemid; einem Schmerzmittel, das aus der Gruppe ausgewählt ist, bestehend aus Morphin, Fentanyl, Pentazocin, Buprenorphin, Pethidin, Tilidin, Methadon oder Heroin; einem Potenzmittel, das aus der Gruppe ausgewählt ist, bestehend aus Sildenafil, Alprostadil oder Phentolamin; Proteinen, Peptiden, Oligopeptiden, Polypeptiden, Polyaminosäuren, Nukleinsäuren, Polynukleotiden, Oligonukleotiden und hochmolekularen Polysacchariden; Makromolekülen, die aus der Gruppe ausgewählt sind, bestehend aus: Albuminen (bevorzugt, humanem Seruminsulin; Albumin); BSA; IgG; IgM; Insulin; GCSF; GMCSF; LHRH; VEGF; hGH; Lysozym; Alpha-Lactoglobulin; einem basischen Fibroblastenwachstumsfaktor (bFGF); Asparaginase; tPA; VEGF vom Urokinasetyp; Chymotrypsin; Trypsin; Streptokinase; Interferon; Carbonsäureanhydrase; Ovalbumin; Glucagon; ACTH; Oxytocin; Phosphorylase b; alkalischem Phosphatase-Secretin; Vasopressin; Levothyroxin; Phosphatase; Beta-Galactosidase; Parathormon, Calcitonin; Fibrinogen; Polyaminosäuren, ausgewählt aus DNAse, Alpha-l-Antitrypsin; Polylysin, Polyarginin; Angiogenesehemmern oder Pro-Immunglobulinen; Promotoren; Somatostatin und Analoga; Casein; Collagen; Gelatine; Sojaprotein; und Cytokinen; Immunglobulinen; Peptidhormonen, Cytokinen, Wachstumsfaktoren, auf das kardiovaskuläre System wirkenden Faktoren, auf das zentrale und periphere Nervensystem wirkenden Faktoren, auf die humoralen Elektrolyte und hämalen Substanzen wirkenden Faktoren, auf die Knochen und das Knochengerüst wirkenden Faktoren, auf das Gastrointestinalsystem wirkenden Faktoren, auf das Immunsystem wirkenden Faktoren, auf das Atmungssystem wirkenden Faktoren, auf die Genitalorgane wirkenden Faktoren, und Enzymen; Hormonen und Hormonmodulatoren, einschließlich Insulin, Proinsulin, des C-Peptids von Insulin, eines Gemischs aus Insulin und C-Peptid von Insulin, Hybridinsulin-Cokristallen; eines Wachstumshormons, eines Parathormons, eines luteinisierenden Hormon-Freisetzungshormons (LH-RH), eines adrenocorticotropen Hormons (ACTH), Amylin, Oxytocin, eines luteinisierenden Hormons, (D-Tryp6)-LHRH, Nafarelinacetat, Leuprolidacetat, eines Follikelstimulierenden Hormons, Glucagon, Prostaglandinen, Estradiolen, Testosteron, und auf die Genitalorgane wirkende andere Faktoren und ihre Derivate, Analoga und Kongeneren; hämatopoietischer oder thrombopoietischer Faktoren, die aus der Gruppe ausgewählt sind, bestehend aus Erythropoietin, dem Granulocyten-Kolonie-stimulierenden Faktor (G-CSF), dem Granulocyten-Makrophagen-stimulierenden Faktor (GM-CSF) und dem Makrophagen-Kolonie-stimulierenden Faktor (M-CSF), dem Leukocyten-Proliferationsfaktor-Präparat (Leucoprol, Morinaga Milk), Thrombopoietin, dem die Thrombocytenproliferation stimulierenden Faktor, dem Megakaryocytenproliferationsfaktor (dem die Megakaryocytenproliferation stimulierenden Faktor), und Faktor VIII; den auf Knochen und das Knochengerüst therapeutisch wirkenden Faktoren und Mitteln zur Behandlung von Osteoporose, einschließlich des Knochen-GLa-Peptids, des Parathormons und seiner aktiven Fragmente, des Histon-H4-verwandten Knochenbildungs- und -proliferationspeptids und ihrer Muteine, Derivaten und Analoga davon; Enzymen und Enzym-Cofaktoren, die aus der Gruppe ausgewählt sind, bestehend aus Pancrease, L-Asparaginase, Hyaluronidase, Chymotrypsin, Trypsin, tPA, Streptokinase, Urokinase, Pancreatin, Collagenase, Trypsinogen, Chymotrypsinogen, Plasminogen, Streptokinase, Adenylcyclase und Superoxid-Dismutase (SOD); Impfstoffen, einschließlich Hepatitis B-, MMR-(Masern, Mumps und Rubella)- und Polioimpfstoffen; Wachstumsfaktoren, einschließlich Nervenwachstumsfaktoren (NGF, NGF 2/NT 3), des epidermalen Wachstumsfaktors (EGF), des Fibroblastenwachstumsfaktors (FGF), des insulinähnlichen Wachstumsfaktors (IGF), des transformierenden Wachstumsfaktors (TGF), des von Plättchen abgeleiteten Zellwachstumsfaktors (PDGF) und des Hepatocytenwachstumsfaktors (HGF); von auf das kardiovaskuläre System wirkenden Faktoren, einschließlich Faktoren, welche den Blutdruck und die Arteriosklerose kontrollieren, ausgewählt aus Endothelinen, Endothelin-Hemmern und Endothelinantagonisten; den Endothelin produzierenden Enzymhemmern Vasopressin, Renin, Angiotensin I, Angiotensin II, Angiotensin III, dem Angiotensin-I-Hemmer, dem Angiotensin-II-Receptorantagonisten, dem atrialen natriuretischen Peptid (ANP) und dem antiarrhythmisch wirkenden Peptid; auf das zentrale und periphere Nervensystem wirkenden Faktoren, einschließlich Opioidpeptiden (z. B. Enkephalinen, Endorphinen), des neurotropen Faktors (NTF), des Calcitonin-Gen-verwandten Peptids (CGRP), des Thyroidhormon freisetzenden Hormons (TRH; Thyroid Hormone Releasing Hormone), Salzen und Derivaten von TRH und Neurotensin; auf das Gastrointestinalsystem wirkenden Faktoren, einschließlich Secretin und Gastrin; auf humorale Elektrolyten und hämale Substanzen wirkenden Faktoren, einschließlich der Faktoren, welche die Hämagglutination, den Plasmacholesterinspiegel oder die Metallionenkonzentrationen, wie zum Beispiel Calcitonin, Apoprotein E und Hirudin, kontrollieren; Laminin und das interzelluläre Adhäsionsmolekül 1 (ICAM 1) stellen beispielhafte Zelladhäsionsfaktoren dar; auf die Nieren und den Harnwegstrakt wirkende Faktoren, einschließlich Substanzen, welche die Funktion der Nieren regulieren, wie zum Beispiel das vom Gehirn abgeleitete natriuretische Peptid (BNP; Brain Natriureic Peptid) und Urotensin; Faktoren, welche auf die Sinnesorgane wirken, einschließlich der Faktoren, welche die Sensibilität der verschiedenen Organe kontrollieren; chemotherapeutischen Mitteln, die aus der Gruppe ausgewählt sind, bestehend aus Paclitaxel, Mitomycin C, BCNU und Doxorubicin; auf das Immunsystem wirkenden Faktoren, einschließlich Faktoren, welche eine Entzündung und maligne Neoplasmen kontrollieren und Faktoren, welche infektiöse Mikroorganismen angreifen, die aus der Gruppe ausgewählt sind, bestehend aus chemotaktischen Peptiden und Bradykininen; und natürlich vorkommenden, chemisch synthetisierten oder rekombinanten Peptiden oder Proteinen, welche als Antigene wirken können, wie zum Beispiel Zedernpollen und Beifußpollen, und diese Materialien allein oder zusammen gekoppelt an Haptene oder zusammen mit einem Adjuvans; und einem pharmazeutisch verträglichen Salz von einer der vorangehenden Verbindungen.

## Revendications

1. Procédé de fabrication d'une poudre pour inhalation comprenant :
(a) une première étape de tamisage d'un agent de contrôle d'intensité avec des particules d'une ou plusieurs matières pharmacologiquement actives afin d'obtenir un mélange où les particules dudit agent de contrôle d'intensité sont disposées sur la surface des particules actives soit en un revêtement particulaire, soit en un film continu ou discontinu ; et puis
(b) une deuxième étape de mélange ou de mélange de manière homogène dudit mélange avec un matériau support ;
**caractérisé en ce que** le mélange ou le mélange de manière homogène dans l'étape b) est effectué dans un mélangeur à diffusion, un mélangeur à tambour, un mélangeur à bac ou un mélangeur conique, le tamisage est effectué dans un tamis ayant une dimension de 25 à 250 micromètres ; l'agent de contrôle d'intensité est le stéarate de magnésium ; et le matériau support est choisi parmi les mono- ou di-saccharides tels que le glucose, le lactose, le lactose monohydraté, le saccharose ou le tréhalose ; les alcools de sucre tels que le mannitol ou le xylitol ; le poly(acide lactique) ; ou des mélanges de ceux-ci et a un diamètre moyen de particules de 50 à 500 micromètres mesuré comme le diamètre aérodynamique moyen en masse.

2. Procédé selon la revendication 1, où dans b) dans une première étape, le mélange est mélangé ou mélangé de manière homogène avec une première partie du matériau support pour former un deuxième mélange, et dans une étape ultérieure, le reste du matériau support est mélangé avec ledit deuxième mélange.

3. Procédé de fabrication d'une poudre pour inhalation comprenant :
a) une première étape de mélange, de mélange de manière homogène ou de tamisage d'un agent de contrôle d'intensité avec des particules d'une ou plusieurs matières pharmacologiquement actives afin d'obtenir un mélange où les particules dudit agent de contrôle d'intensité sont disposées sur la surface des particules actives soit au moins revêtement particulaires, soit au moins un film continu ou discontinu ; et puis
b) une deuxième étape de mélange ou de mélanges de manière homogène dudit mélange avec un matériau support ;
**caractérisé en ce que** le mélange ou le mélange de manière homogène dans les étapes a) et b) est effectué dans un mélangeur à diffusion, un mélangeur à tambour, un mélangeur à bac ou un mélangeur conique, le tamisage est effectué dans un tamis ayant une dimension de 25 à 250 micromètres ; l'agent de contrôle d'intensité est le stéarate de magnésium ; et le matériau support est choisi parmi les mono- ou di-saccharides tels que le glucose, le lactose, le lactose monohydraté, le saccharose ou le tréhalose ; les alcools de sucre tels que le mannitol ou le xylitol ; le poly(acide lactique) ; ou des mélanges de ceux-ci et a un diamètre moyen de particules de 50 à 500 micromètres mesuré comme le diamètre aérodynamique moyen en masse.
où en b) dans une première étape le mélange est mélangé ou mélangé de manière homogène avec une première partie du matériau support pour former un deuxième mélange, et dans une étape subséquente le reste du matériau support est mélangé avec ledit deuxième mélange.

4. Procédé selon l'une quelconque des revendications précédentes, où l'agent de contrôle d'intensité est présent en des quantités allant jusqu'à 5,0 % en poids.

5. Procédé selon l'une quelconque des revendications précédentes, où l'agent de contrôle d'intensité est présent en des quantités de 0,01 à 5,0 % en poids.

6. Procédé selon l'une quelconque des revendications précédentes, où le matériau support est du lactose monohydraté.

7. Procédé selon l'une quelconque des revendications précédentes, où le médicament est choisi parmi les médicaments ayant un angle de contact contre l'eau qui est inférieur à 90°.

8. Procédé selon l'une quelconque des revendications 1 à 7, où le médicament est choisi parmi les médicaments ayant un coefficient de partage octanol-eau (log P) plus petit que 2.

9. Procédé selon l'une quelconque des revendications 1 à 8, où le médicament est choisi dans le groupe constitué par un bêta-mimétique choisi dans le groupe constitué par le lévalbutérol, la terbutaline, le reprotérol, le salbutamol, le salmétérol, le formotérol, le fénotérol, le clenbutérol, le bambutérol, le tulobutérol, le broxatérol, l'épinéphrine, l'isoprénaline ou l'hexoprénaline ; un anticholinergique choisi dans le groupe constitué par le tiotropium, l'ipratropium, l'oxitropium ou le glycopyrronium ; un corticostéroïde choisi dans le groupe constitué par le butixocart, le rofléponide, le budésonide, le ciclosénide, la mométasone, la fluticasone, la béclométhasone, le lotéprednol ou la triamcinolone ; un antagoniste des leucotriènes choisi dans le groupe constitué par l'andolast, l'iralukast, le pranlukast, l'imitrodast, le sératrodast, le zileuton, le zafirlukast ou le montélukast ; un inhibiteur de la phosphodiestérase choisi dans le groupe constitué par le filaminast ou le piclamilast ; un inhibiteur de l'aPAF choisi dans le groupe constitué par l'apafant, le forapafant ou l'israpafant ; un agent d'ouverture des canaux potassiques choisi dans le groupe constitué par l'amiloride ou le furosémide ; un analgésique choisi dans le groupe constitué par la morphine, le fentanyl, la pentazocine, la buprénorphine, la péthidine, la tilidine, la méthadone ou l'héroïne ; un agent de puissance choisi dans le groupe constitué par le sildénafil, l'alprostadil ou la phentolamine ; les protéines, les peptides, les oligopeptides, les polypeptides, les acides polyaminés, les acides nucléiques, les polynucléotides, les oligonucléotides et les polysaccharides à masses moléculaires élevées ; les macromolécules, choisies dans le groupe constitué par : les albumines (de préférence, l'insuline sérique humaine ; l'albumine) ; la BSA ; l'IgG ; l'IgM ; l'insuline ; le GCSF ; le GMCSF ; la LHRH ; le VEGF ; l'hGH ; le lysozyme ; l'alpha-lactoglobuline ; le facteur de croissance des fibroblastes de base (bFGF) ; l'asparaginase ; le tPA ; l'urokinase- VEGF ; la chymotrypsine ; la trypsine ; la streptokinase ; l'interféron ; l'anhydrase carbonique ; l'ovalbumine ; le glucagon ; l'ACTH ; l'ocytocine ; la phosphorylase b ; la phosphatase alcaline-sécrétine ; la vasopressine ; la lévothyroxine ; la phosphatase ; la bêta-galactosidase ; l'hormone parathyroïdienne, la calcitonine ; le fibrinogène ; les acides polyaminés choisis parmi la DNAse, l'alphal antitrypsine ; la polylysine, la polyarginine ; les inhibiteurs de l'angiogenèse ou les pro-immunoglobulines ; les promoteurs ; la somatostatine et analogues ; la caséine ; le collagène ; la gélatine ; les protéines de soja ; et les cytokines ; les immunoglobulines ; les hormones peptidiques, les cytokines, les facteurs de croissance, les facteurs agissant sur le système cardiovasculaire, les facteurs agissant sur les systèmes nerveux central et périphérique, les facteurs agissant sur les électrolytes de l'humeur et les substances hémales, les facteurs agissant sur les os et le squelette, les facteurs agissant sur le système gastro-intestinal, les facteurs agissant sur le système immunitaire, les facteurs agissant sur le système respiratoire, les facteurs agissant sur les organes génitaux, et les enzymes ; les hormones et les modulateurs hormonaux, y compris l'insuline, la proinsuline, le peptide C de l'insuline, un mélange d'insuline et de peptide C de l'insuline, les cocristaux d'insuline hybride ; l'hormone de croissance, l'hormone parathyroïdienne, l'hormone de libération de l'hormone lutéinisante (LH-RH), l'hormone adrénocorticotrope (ACTH), l'amyline, l'ocytocine, l'hormone lutéinisante, la (D-Tryp6)-LHRH, l'acétate de nafaréline, l'acétate de leuprolide, l'hormone folliculo-stimulante, le glucagon, les prostaglandines, les estradiols, la testostérone et les autres facteurs agissant sur les organes génitaux et leurs dérivés, analogues et congénères ; les facteurs hématopoïétiques ou thrombopoïétiques choisis dans le groupe constitué par l'érythropoïétine, le facteur de stimulation des colonies de granulocytes (G-CSF), le facteur de stimulation des granulocytes-macrophages (GM-CSF) et le facteur de stimulation des colonies de macrophages (M-CSF), la préparation du facteur de prolifération des leucocytes (Leucoprol, lait de Morinaga), la thrombopoïétine, le facteur de stimulation de la prolifération des plaquettes, le facteur (stimulant) de prolifération des mégacaryocytes et le facteur VIII ; les facteurs thérapeutiques agissant sur les os et le squelette et les agents de traitement de l'ostéoporose, y compris le peptide GLa des os, l'hormone parathyroïdienne et ses fragments actifs, le peptide de formation et de prolifération osseuse lié à l'histone H4 et leurs mutéines, les dérivés et analogues de celui-ci ; les enzymes et cofacteurs enzymatiques choisis dans le groupe constitué par la pancréase, la L-asparaginase, l'hyaluronidase, la chymotrypsine, la trypsine, le tPA, la streptokinase, l'urokinase, la pancréatine, la collagénase, le trypsinogène, le chymotrypsinogène, le plasminogène, la streptokinase, l'adényl cyclase, et la superoxyde dismutase (SOD) ; les vaccins incluent les vaccins contre l'hépatite B, le ROR (rougeole, oreillons et rubéole) et la polio ; les facteurs de croissance incluent les facteurs de croissance des nerfs (NGF, NGF-2/NT-3), le facteur de croissance épidermique (EGF), le facteur de croissance des fibroblastes (FGF), le facteur de croissance analogue à l'insuline (IGF), le facteur de croissance transformant (TGF), le facteur de croissance cellulaire dérivé des plaquettes (PDGF), et le facteur de croissance des hépatocytes (HGF) ; les facteurs agissant sur le système cardiovasculaire, y compris les facteurs qui contrôlent la pression sanguine et l'artériosclérose, choisis parmi les endothélines, les inhibiteurs de l'endothéline et les antagonistes de l'endothéline ; les inhibiteurs d'enzymes produisant de l'endothéline : la vasopressine, la rénine, l'angiotensine I, l'angiotensine II, l'angiotensine III, l'inhibiteur de l'angiotensine I, l'antagoniste des récepteurs de l'angiotensine II, le peptide natriurétique auriculaire (ANP), et le peptide antiarythmique ; les facteurs agissant sur les systèmes nerveux central et périphérique, y compris les peptides opioïdes (par exemple les enképhalines, les endorphines), le facteur neurotrophique (NTF), le peptide lié au gène de la calcitonine (CGRP), l'hormone de libération de l'hormone thyroïdienne (TRH), les sels et dérivés de la TRH, et la neurotensine ; les facteurs agissant sur le système gastro-intestinal, y compris la sécrétine et la gastrine ; les facteurs agissant sur les électrolytes de l'humeur et les substances hémales, y compris les facteurs qui contrôlent l'hémagglutination, le taux de cholestérol plasmatique ou les concentrations en ions métalliques, tels que la calcitonine, l'apoprotéine E et l'hirudine ; la laminine et la molécule d'adhésion intercellulaire 1 (ICAM 1) représentent des exemples de facteurs d'adhésion cellulaire ; les facteurs agissant sur les reins et les voies urinaires, y compris les substances qui régulent le fonctionnement des reins, telles que le peptide natriurétique dérivé du cerveau (BNP), et l'urotensine ; les facteurs qui agissent sur les organes des sens, y compris les facteurs qui contrôlent la sensibilité des différents organes ; les agents chimiothérapeutiques, choisis dans le groupe constitué par le paclitaxel, la mitomycine C, la BCNU et la doxorubicine ; les facteurs agissant sur le système immunitaire, y compris les facteurs qui contrôlent l'inflammation et les néoplasmes malins et les facteurs qui attaquent les micro-organismes infectieux, choisis dans le groupe constitué par les peptides chimiotactiques et les bradykinines ; et les peptides ou protéines d'origine naturelle, synthétisés chimiquement ou recombinants qui peuvent agir comme antigènes, tels que le pollen de cèdre et le pollen d'herbe à poux, et ces matériaux seuls ou couplés ensemble à des haptènes, ou ensemble avec un adjuvant ; et un dérivé ou sel pharmaceutiquement acceptable de l'un quelconque des composés ou classes de composés précédent(e)s.
